# NEUE EUROPÄISCHE PATENTSCHRIFT

(11) **EP 1 502 617 B2**
(45) Veröffentlichungstag und Bekanntmachung des Hinweises auf die Entscheidung über den Einspruch: **27.04.2022**
(45) Hinweis auf die Patenterteilung: 27.03.2013
(21) Anmeldenummer: 04018070.5
(22) Anmeldetag: 30.07.2004
(51) Int. Cl.: A61M 5/32

(54) **Nadelschutz für eine Glasspritze**
Needle protector for a glass syringe
Protecteur d'aiguille pour une seringue à vidre

(30) Priorität: 01.08.2003 EP 03017423
(43) Veröffentlichungstag der Anmeldung: 02.02.2005
(73) Patentinhaber: Gerresheimer Bünde GmbH, 32257 Bünde (DE)
(72) Erfinder: Stohlmann, Erhard, 32257 Bünde (DE); Wittland, Frank, 32130 Enger (DE)
(74) Vertreter: Philipp, Matthias

(56) Entgegenhaltungen:
- EP-A- 0 876 824
- EP-A2- 0 876 824
- WO-A-02/074367
- WO-A2-02/074367
- FR-A- 2 777 787
- FR-A1- 2 777 787
- US-A- 4 964 866
- US-A- 4 986 818
- US-A1- 2002 062 108
- US-A1- 2004 140 285
- US-B1- 6 551 286
- US-B1- 6 551 286
- Walter Michaeli, Einführung in die Kunstoffverarbeitung, Carl Hanser Verlag, 1992, Seiten 127-129

## Beschreibung

Die Erfindung betrifft einen Nadelschutz für eine Glasspritze, der einen Verschlußstopfen, ein im wesentlichen die Form eines Hohlzylinders aufweisendes Halteelement mit einer Öffnung zum Aufnehmen eines Abschnitts eines Spritzenkörpers der Glasspritze und ein zugeordnetes, im wesentlichen die Form eines Hohlzylinders aufweisendes Schutzelement aufweist, wobei der Verschlußstopfen und das Halteelement aus einem elastischen Material und das Schutzelement aus einem härteren Material gebildet sind.

Es ist bekannt, daß Glasspritzen mit Nadeln, die im allgemeinen mit dem Spritzenkörper beispielsweise durch Klebemittel dauerhaft verbunden sind, mit einem aufschiebbaren Nadelschutz aus elastischem Material, wie Gummi, verschlossen werden können. Dieser Nadelschutz umschließt die Nadel und kann problemlos abgezogen werden. Ein derartiger Nadelschutz wird unter anderem in US 3,865,236 beschrieben.

Probleme treten auf, wenn die Glasspritze unterschiedlichen Temperaturen oder Luftdrücken ausgesetzt wird. In der Regel werden Glasspritzen, nachdem sie mit einer Flüssigkeit, die beispielsweise pharmazeutische Wirkstoffe enthält, gefüllt und mit dem bekannten Nadelschutz verschlossen worden sind, sterilisiert.

Dabei werden sie hohen Temperaturen, zum Beispiel 121°C oder 134°C und einem Vakuum ausgesetzt, so daß sich der elastische Nadelschutz aufbläht. Dies führt häufig zu einer Lockerung des Nadelschutzes und zu einem Abrutschen von der Glasspritze. So kommt es bei der Sterilisation oft zu einem Abspringen des Nadelschutzes von der Glasspritze (engl. pop-off effect).

Aus der US 6,551,286 B1 ist ein Nadelschutz gemäß dem Oberbegriff des Anspruchs 1 bekannt.

Es ist die Aufgabe der vorliegenden Erfindung, einen Nadelschutz anzugeben, bei dem die oben beschriebene Lockerung verhindert und die Zuführung von sterilisierenden Gasen durch Permeation verbessert wird. Weiterhin soll ein Nadelschutz angegeben werden, bei dem eine nachträgliche Manipulation der Spritzenfüllung sofort erkennbar ist.

Diese Aufgabe wird bei einem Nadelschutz der aufgezeigten Gattung gemäß Anspruch 1 dadurch gelöst, daß der Verschlußstopfen und das elastische Halteelement aus gasdurchlässigem Material gebildet sind, wobei das Gas zur Sterilisation bestimmt ist.

Gemäß der Erfindung sind weiterhin bei einteiliger Ausbildung des Verschlußstopfens, des Halteelements und des Schutzelements zwischen dem Schutzelement und dem Verschlußstopfen längsverlaufende Gaszuführungsschlitze angeordnet, wobei das Schutzelement an seinem Spritze abgewandten Ende offen ist. Es besteht die Möglichkeit, unterhalb der Gaszuführungsschlitze das Halteelement durch das Schutzelement bis zur äußeren Begrenzung des Schutzelements zu führen, wodurch eine zusätzliche formschlüssige Verbindung hergestellt und ein Gasdurchgang ermöglicht wird. Das Halteelement kann außenwandige Stufen aufweisen. Vorteilhafterweise weist das Halteelement ein Rastelement auf, das mit einem Rastelement des Spritzenkörpers verrastbar ist, wobei das Rastelement des Halteelements durch eine nach innen weisende Ringwulst und das Rastelement des Spritzenkörpers durch eine ringförmige Nut gebildet ist.

Zweckmäßige Ausgestaltungen der Erfindung sind in den übrigen Unteransprüchen aufgezeigt.

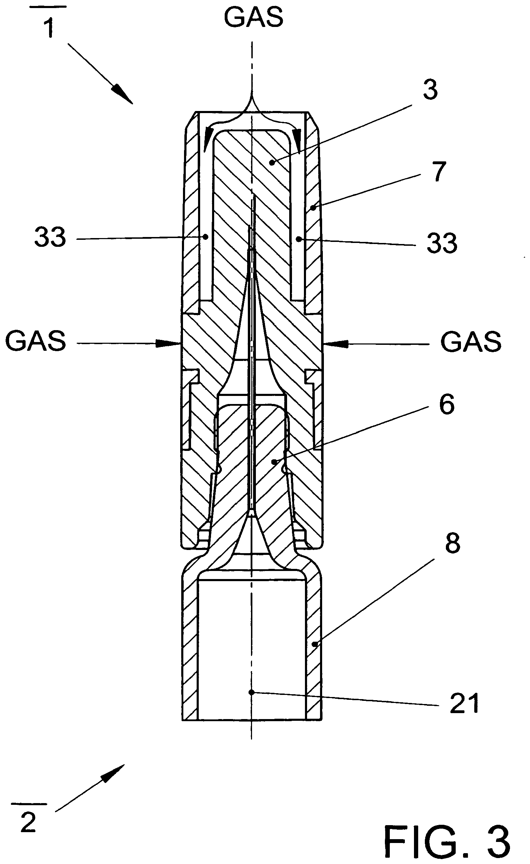

Ein Ausführungsbeispiel der Erfindung wird nachfolgend anhand der Figur 3 erläutert. Es zeigen:
- Fig. 1: einen Vertikalschnitt durch einen Nadelschutz mit Verschlußstopfen, der auf eine Glasspritze aufgeschoben ist;
- Fig. 2: einen Vertikalschnitt durch eine weitere Ausführungsform eines Nadelschutzes, der auf eine Glasspritze aufgeschoben ist;
- Fig. 3: einen Vertikalschnitt durch eine Ausführungsform eines erfindungsgemäßen Nadelschutzes, der auf eine Glasspritze aufgeschoben ist und
- Fig. 4: einen Vertikalschnitt durch einem Nadelschutz, der auf eine Glasspritze aufgeschoben ist.

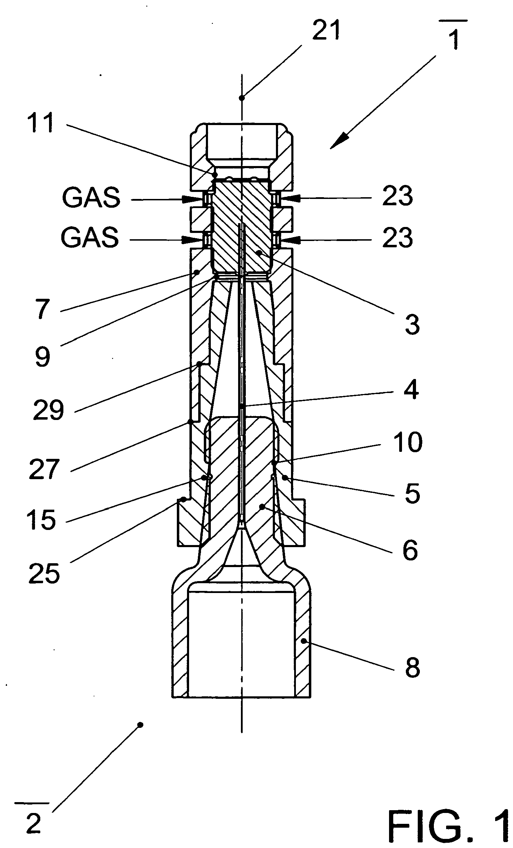

Nach Fig. 1 weist der Nadelschutz 1 ein Halteelement 5, ein Schutzelement 7 und einen Verschlussstopfen 3 auf. Das Halteelement 5 besteht aus einem elastischen Material, beispielsweise aus einem thermoplastischen, vulkanisierten Elastomer (TPE-V). Es ist im wesentlichen in der Form eines Hohlzylinders ausgebildet, der an seinen Außenwänden Stufen 25, 27, 29 aufweist. Im aufgeschobenen Zustand nimmt das zylinderförmige Halteelement 5 einen Abschnitt 6 des Spritzenkörpers 8 der Glasspritze 2 auf. Das Halteelement 5 weist unterschiedliche Außendurchmesser auf, wobei der Außendurchmesser des der Glasspritze 2 zugewandten Endabschnitts am größten und der Außendurchmesser des anderen gegenüberliegenden Endabschnittes am kleinsten ist. Der Außendurchmesser des zuletzt genannten Endabschnittes des Halteelementes 5 ist so gewählt, daß er von dem Schutzelement 7 aufgenommen werden kann.

Im aufgeschobenen Zustand hält das Halteelement 5 aufgrund seiner Elastizität den Nadelschutz 1 auf der Glasspritze 2 und dichtet den Innenraum des Nadelschutzes 1 ab.

Das Schutzelement 7 besteht aus einem formstabilen Material, wie beispielsweise Polypropylen, das im Vergleich zu dem Halteelement 5 und zu dem Verschlussstopfen 3 kaum elastisch ist. Das Schutzelement 7 ist im wesentlichen als Hohlzylinder ausgebildet, der nach dem Aufsetzen des Nadelschutzes 1 auf die Glasspritze 2 die Nadel 4 umschließt. Das zylinderförmige Schutzelement 7 kann entlang seiner Längsachse 21 unterschiedliche Innendurchmesser aufweisen, wobei der Innendurchmesser des dem Halteelement 5 zugewandten Endabschnittes so bemessen ist, daß der entsprechende Endabschnitt des Halteelementes 5 aufgenommen werden kann. Der andere Endabschnitt des Schutzelementes 7 weist einen solchen Innendurchmesser auf, dass der Verschlussstopfen 3 aufnehmbar ist. Das Schutzelement 7 und das Halteelement 5 sind einteilig mittels Zwei-Komponenten-Spritzgießverfahren hergestellt und dauerhaft miteinander verbunden, in dem das Schutzelement 7 aus PP und das Halteelement aus TPE-V gefertigt sind. Das Schutzelement 7 ist an seinem der Spritze abgewandten Ende offen, könnte aber auch geschlossen sein. Es weist zwei Halterungen 9, 11 auf, die den Verschlussstopfen 3 an einer vorbestimmten Position halten. Im Bereich des Verschlußstopfens 3 weist das Schutzelement 7 Öffnungen 23 auf, die in diesem Beispiel länglich ausgebildet sind und quer zur Längsachse 21 des im wesentlichen zylinderförmigen Schutzelementes 7 verlaufen. Die Öffnungen 23 haben die Aufgabe, sterilisierende Gase durch Permeation durch den Verschlußstopfen 3 eindringen zu lassen.

Der Verschlußstopfen 3 besteht aus einem elastischen pharmazeutischen Gummi, z. B. SBR (Styrol Butadien Rubber) oder NBR (Natural Butadien Rubber) oder BIIR (Brom Isobutene Isoprene Rubber) oder CIIR (Chlor Isobutene Isoprene Rubber) und ist gasdurchlässig. Der Verschlußstopfen 3 ist zylinderförmig ausgebildet und weist einen Außendurchmesser auf, der im wesentlichen gleich dem Innendurchmesser des dem Spritzenkörper 8 abgewandten Endabschnittes des Schutzelementes 7 ist. Im aufgesetzten Zustand dringt die Nadel 4 der Glasspritze 2 in den Verschlußstopfen 3 ein, so daß die Nadel 4 verschlossen ist.

Da der erfindungsgemäße Nadelschutz 1 zu einem großen Teil aus dem Schutzelement 7 besteht, das im Vergleich zu dem Halteelement 5 und dem Verschlußstopfen 3 kaum elastisch ist, tritt eine unerwünschte Lokkerung des Nadelschutzes 1 aufgrund von unterschiedlichen Temperaturen oder Luftdrücken kaum auf.

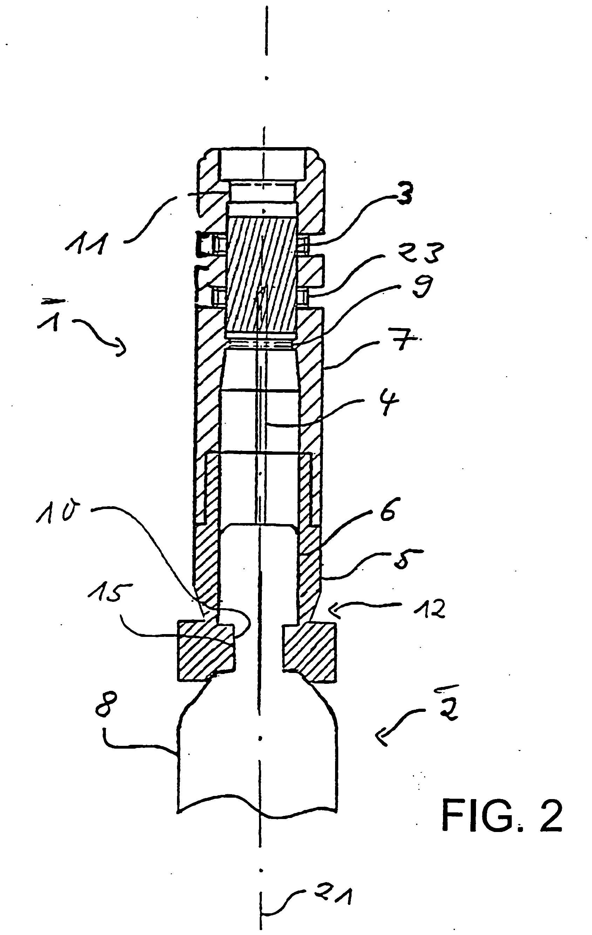

Ein weiteres Beispiel ist in Fig. 2 dargestellt. Auch hier besteht der Nadelschutz 1 aus einem Halteelement 5, einem Schutzelement 7 und einem Verschlußstopfen 3. Während das Schutzelement 7 und der Verschlußstopfen 3 im Vergleich zur ersten Ausführungsform unverändert sind, weist das Halteelement 5 hier ein Rastelement 15 auf, das in dem in Fig. 2 gezeigten aufgesetzten Zustand mit einem Rastelement 10 des Spritzenkörpers 8 unlösbar verrastet ist. Das Rastelement 15 des Halteelements 5 ist eine Ringwulst und das Rastelement 10 des Spritzenkörpers 8 eine ringförmige Erweiterung oder Vertiefung. Auf diese Art ist das Halteelement 5 mit dem Spritzenkörper 8 dauerhaft verbunden. In anderen Ausführungsbeispielen könnte das Halteelement 5 auch durch andere Verbindungsmittel, zum Beispiel durch Klebemittel, unlösbar mit dem Spritzenkörper 8 verbunden sein. Das Halteelement 5 weist angrenzend an die dem Spritzenkörper 8 abgewandte Seite des Rastelements 15 eine Sollbruchstelle 12 auf, die als umlaufende Querschnittsschwächung ausgebildet ist. Die Sollbruchstelle 12 kann auch auf eine andere Art ausgebildetsein, beispielsweise durch Schlitze. Aufgabe der Sollbruchstelle ist die unmittelbare Sichtbarmachung einer Manipulation an der Spritzenfüllung. Beim Abnehmen des Nadelschutzes 1 gemäß dem in Fig. 2 gezeigten Beispiel wird das Rastelement 15 an der Sollbruchstelle 12 vom dem Halteelement 5 abgetrennt und verbleibt am Spritzenkörper 8. Wird der auf diese Weise beschädigte Nadelschutz 1 wieder auf die Glasspritze 2 aufgeschoben, so ist das vorherige Abnehmen des Nadelschutzes 1 an dem an der Sollbruchstelle 12 durchbrochenen Halteelement 5 erkennbar.

In Fig. 3 ist eine Ausführungsform der Erfindung dargestellt. Im Vergleich zu den Beispielen nach den Fig. 1 und 2 sind der Verschlußstopfen 3, das Halteelement 5 und das Schutzelement 7 einteilig im Zwei-Komponenten-Spritzgießverfahren hergestellt, wobei der Verschlußstopfen 3 und das Halteelement 5 aus einem identischen elastischen Material und das Schutzelement 7 aus einem härteren Material gebildet sind. Die Gaszuführung erfolgt nach Fig. 3 über längsverlaufende Gaszuführungsschlitze 33, die zwischen dem Schutzelement 7 und dem Verschlußstopfen 3 verlaufen.

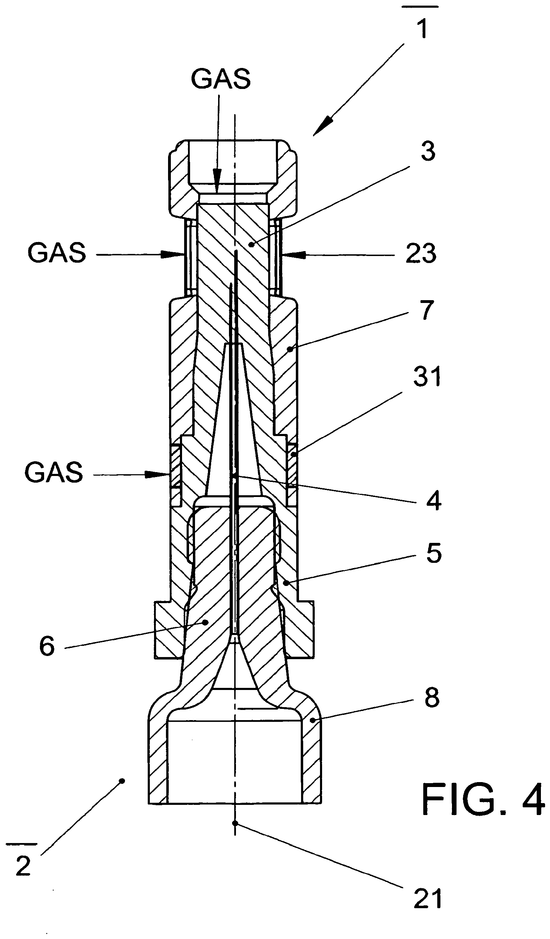

Im Gegensatz zur Ausführung nach Fig. 3 erfolgt nach Fig. 4 die Gaszuführung nicht durch längsverlaufende Schlitze 33, sondern über im oberen Bereich des Schutzelements 7 angeordnete quer zur Längsachse verlaufende Öffnungen 23 und zusätzlich über quer zur Längsachse 21 verlaufende Durchbrüche 31 in dem Schutzelement 7 in Höhe des unteren Bereichs der Nadel 4.

Das Schutzelement 7 kann außenwandige Rippen aufweisen, die beispielsweise parallel zur Längsachse 21 des Schutzelementes 7 angeordnet sein können. Die Rippen verbessern die Griffigkeit des Schutzelements 7.

### Aufstellung der Bezugszeichen:

- 1: Nadelschutz
- 2: Glasspritze
- 3: Verschlußstopfen
- 4: Nadel
- 5: Halteelement
- 6: Abschnitt des Spritzenkörpers
- 7: Schutzelement
- 8: Spritzenkörper
- 9: Halterungen
- 10: Rastelement von 8
- 11: Halterung
- 12: Sollbruchstelle
- 15: Rastelement von 5
- 21: Längsachse
- 23: Öffnungen
- 25: außenwandige Stufen von 5
- 27: außenwandige Stufen von 5
- 29: außenwandige Stufen von 5
- 31: quer zur Längsachse verlaufende Durchbrüche
- 33: längsverlaufende Gaszuführungsschlitze

## Patentansprüche

1. Nadelschutz für eine Glasspritze (2), der einen Verschlussstopfen (3), ein im Wesentlichen die Form eines Hohlzylinders aufweisendes Halteelement (5) mit einer Öffnung zum Aufnehmen eines Abschnitts (6) eines Spritzenkörpers (8) der Glasspritze (2) und ein die Form eines Hohlzylinders aufweisendes, das Halteelement (5) übergreifendes Schutzelement (7) aufweist, wobei der Verschlussstopfen (3), das Halteelement (5) und das Schutzelement (7) einteilig im Zwei-Komponenten-Spritzgießverfahren hergestellt sind, wobei Verschlussstopfen und Halteelement aus einem identischen elastischen, für ein zur Sterilisation bestimmtes Gas durchlässigen Material und das Schutzelement (7) aus einem härteren Material gebildet sind, **dadurch gekennzeichnet, dass** zwischen dem Verschlussstopfen (3) und dem Schutzelement (7) längsverlaufende, Schlitze (33) für den Zutritt von zur Sterilisation bestimmtem Gas gebildet sind, und dazu dass Schutzelement an seinem der Spritze (2) abgewandten Ende offen ist.

2. Nadelschutz nach Anspruch 1, **dadurch gekennzeichnet, dass** das Halteelement (5) aus thermoplastischem Elastomer TPE-V und das Schutzelement (7) aus thermoplastischem Kunststoff PP besteht, um eine dauerhafte Verbindung einzugehen.

3. Nadelschutz nach einem der vorangehenden Ansprüche, **dadurch gekennzeichnet, dass** unterhalb der Gaszuführungsschlitze (33) das Halteelement (5) partiell durch das Schutzelement (7) bis zur äußeren Begrenzung des Schutzelements (7) geführt ist.

4. Nadelschutz nach einem der vorangehenden Ansprüche, **dadurch gekennzeichnet, dass** das Halteelement (5) außenwandige Stufen (25, 27, 29) aufweist.

5. Nadelschutz nach einem der vorangehenden Ansprüche, **dadurch gekennzeichnet, dass** das Halteelement (5) ein Rastelement (15) aufweist, das mit einem Rastelement (10) des Spritzenkörpers (8) verrastbar ist.

6. Nadelschutz nach Anspruch 5, **dadurch gekennzeichnet, dass** das Rastelement (15) des Halteelements (5) durch eine nach innen weisende Ringwulst und das Rastelement (10) des Spritzenkörpers (8) durch eine ringförmige Erweiterung oder Vertiefung gebildet ist.

7. Nadelschutz nach Anspruch 5 oder 6, **dadurch gekennzeichnet, dass** das Rastelement des Halteelements (5) beim Entfernen des Nadelschutzes (1) von der Glasspritze (2) von dem Halteelement (5) abtrennbar ist.

8. Nadelschutz nach einem der vorangehenden Ansprüche, **dadurch gekennzeichnet, dass** das Halteelement (5) zumindest eine Sollbruchstelle (12) aufweist.

9. Nadelschutz nach Anspruch 8, **dadurch gekennzeichnet, dass** die Sollbruchstelle (12) umlaufend ausgebildet ist.

10. Nadelschutz nach Anspruch 8 oder 9, **dadurch gekennzeichnet, dass** die Sollbruchstelle (12) an das Rastelement des Halteelements (5) angrenzt.

## Claims

1. Needle protection unit for a glass syringe (2), which comprises a closure element (3), a retaining element (5) having essentially the form of a hollow cylinder with an aperture for receiving a section (6) of a syringe body (8) of the glass syringe (2), and a protective element (7) having the form of a hollow cylinder, straddling the retaining element (5), wherein the closure element (3), the retaining element (5) and the protective element (7) are made as one piece in a two-component injection moulding process, closure element and retaining element being formed from an identical elastic material, letting through a gas used for sterilization and the protective element (7) from a harder material, **characterized in that** lengthwise running slots (33) are formed between the closure element (3) and the protective element (7) in order to admit gas used for sterilization and for this purpose the protective element is open at its end facing away from the syringe (2).

2. Needle protection unit according to claim 1, **characterized in that** the retaining element (5) is made of thermoplastic TPE-V elastomer and the protective element (7) is made of thermoplastic PP polymer, in order to form a permanent bond.

3. Needle protection unit according to one of the preceding claims, **characterized in that** below the gas feed slots (33) the retaining element (5) is partially guided through the protective element (7) as far es the external boundary of the protective element (7).

4. Needle protection unit according to one of the preceding claims, **characterized in that** the retaining element (5) has outer wall steps (25, 27, 29).

5. Needle protection unit according to one of the preceding claims, **characterized in that** the retaining element (5) has a clip element (15), which can snap into a latch element (10) of the syringe body (8).

6. Needle protection unit according to claim 5, **characterized in that** the clip element (15) of the retaining element (5) is formed by an inwardly-directed ring bulge and the latch element (10) of the syringe body (8) by a circular extension or recess.

7. Needle protection unit according to claim 5 or 6, **characterized in that** the clip element of the retaining element (5) can be separated from the retaining element (5) when removing the needle protection unit (1) from the glass syringe (2).

8. Needle protection unit according to one of the preceding claims, **characterized in that** the retaining element (5) has at least one predetermined breaking point (12).

9. Needle protection unit according to claim 8, **characterized in that** the predetermined breaking point (12) is formed circumferentially.

10. Needle protection unit according to claim 8 or 9, **characterized in that** the predetermined breaking point (12) is adjacent to the clip element of the retaining element (5).

## Revendications

1. Unité de protection d'aiguille pour une seringue en verre (2), qui comporte un élément de fermeture (3), un élément de retenue (5) ayant essentiellement la forme d'un cylindre creux avec une ouverture à des fins de réception d'une section (6) d'un corps de seringue (8) de la seringue en verre (2) et un élément de protection (7) ayant la forme d'un cylindre creux, encadrant l'élément de retenue (5), dans lequel l'élément de fermeture (3), l'élément de retenue (5) et l'élément de protection (7) sont réalisés d'une seule pièce au cours du procédé de moulage par injection à deux composants, l'élément de fermeture et l'élément de retenue étant formés à partir d'un matériau souple identique, laissant passer un gaz utilisé à des fins de stérilisation et l'élément de protection (7) à partir d'un matériau plus dur, **caractérisé en ce que** des fentes s'étendant dans le sens de la longueur (33) sont formées entre l'élément de fermeture (3) et l'élément de protection (7) afin d'admettre le gaz utilisé à des fins de stérilisation et dans ce but l'élément de protection est ouvert au niveau de son extrémité orientée à l'opposé en provenance de la seringue (2).

2. Unité de protection d'aiguille selon la revendication 1, **caractérisée en ce que** l'élément de retenue (5) est réalisé en élastomère TPE-V thermoplastique et l'élément de protection (7) en polymère PP thermoplastique, afin de former un assemblage permanent.

3. Unité de protection d'aiguille selon l'une quelconque des revendications précédentes, **caractérisée en ce que**, sous les fentes d'alimentation en gaz (33), l'élément de retenue (5) est partiellement guidé au travers de l'élément de protection (7) jusqu'à la limite externe de l'élément de protection (7).

4. Unité de protection d'aiguille selon l'une quelconque des revendications précédentes, **caractérisée en ce que** l'élément de retenue (5) a des gradins de paroi extérieure (25, 27, 29).

5. Unité de protection d'aiguille selon l'une quelconque des revendications précédentes, **caractérisée en ce que** l'élément de retenue (5) a un élément d'attache (15), qui peut s'enclencher dans un élément de verrouillage (10) du corps de seringue (8).

6. Unité de protection d'aiguille selon la revendication 5, **caractérisée en ce que** l'élément d'attache (15) de l'élément de retenue (5) est formé par un renflement annulaire dirigé vers l'intérieur et l'élément de verrouillage (10) du corps de seringue (8) par un évidement ou prolongement circulaire.

7. Unité de protection d'aiguille selon la revendication 5 ou la revendication 6, **caractérisée en ce que** l'élément d'attache de l'élément de retenue (5) peut être séparé de l'élément de retenue (5) lors du retrait de l'unité de protection d'aiguille (1) de la seringue en verre (2).

8. Unité de protection d'aiguille selon l'une des revendications précédentes, **caractérisée en ce que** l'élément de retenue (5) a au moins un point de cassure prédéterminé (12).

9. Unité de protection d'aiguille selon la revendication 8, **caractérisée en ce que** le point de cassure (12) est formé dans le sens de la circonférence.

10. Unité de protection d'aiguille selon la revendication 8 ou la revendication 9, **caractérisée en ce que** le point de cassure (12) est adjacent à l'élément d'attache de l'élément de retenue (5).
